# EUROPEAN PATENT SPECIFICATION

(11) **EP 4 490 498 B1**
(45) Date of publication and mention of the grant of the patent: **06.05.2026**
(21) Application number: 23711796.5
(22) Date of filing: 07.03.2023
(51) Int. Cl.: G01N 27/02, G01N 27/06, G01N 27/74, G01N 33/02, G01N 33/04, G01N 33/14, G01N 35/00, B01F 31/441, B01F 33/453, B01F 35/21, B01F 35/50, B01F 35/71, G01N 11/00, G01N 11/12

(54) **MEASURING SYSTEM FOR FOODSTUFFS**
MESSSYSTEM FÜR LEBENSMITTEL
SYSTÈME DE MESURE DESTINÉ AUX PRODUITS ALIMENTAIRES

(30) Priority: 11.03.2022 NL 2031245
(43) Date of publication of application: 15.01.2025
(73) Proprietor: Lanvi Patent B.V., 3147 PB Maassluis (NL)
(72) Inventor: VAN HALSEMA, Frans Emo Diderik, 3147 PB Maassluis (NL)
(74) Representative: Octrooibureau Van der Lely N.V.
(86) International application number: PCT/IB2023/052133
(87) International publication number: WO 2023/170569

(56) References cited:
- WO-A1-2021/066646
- US-A- 6 166 551
- US-A1- 2009 221 431
- US-A1- 2018 008 080

## Description

The present invention concerns a measuring system for automatically determining and/or monitoring a quality of a foodstuff mixture, and comprising at least one product holder with a longitudinal direction for receiving a fluid or viscous foodstuff, a housing with a receiving space for receiving the at least one product holder, a sensor device for measuring a parameter value of the foodstuff in the product holder, and a control device for controlling the measuring system and processing the measured parameter values, furthermore comprising a mixing device which comprises a mixing body arranged in the product holder and comprising a magnetisable or magnetic material, and a displacement device arranged outside the product holder for displacing the mixing body along the longitudinal direction.

Such a measuring system is known from document WO2021066646-A1, and serves inter alia for reliably mixing foodstuffs in slender product holders.

One disadvantage of the known measuring system however is that it is not suitable for many types of measurement, in particular not if an additive must be added to the foodstuff. For this, the product holder must be opened by an operator, the additive added and the product holder closed again. Such actions are often carried out only some time after the start of measurements, for example after setting a specific temperature in the product holder, after maturing of the foodstuff etc. This sometimes makes the performance of measurements awkward or requires complex planning. In any case, the human action, which may lead to errors, can at least reduce reliability.

It is an object of the present invention to provide a measuring system of the specified type which no longer has the above disadvantages, or at least only to a lesser extent.

The invention achieves this object with a measuring system according to claim 1.

The invention here uses the understanding that the mixing body may be used to automatically output the additive to the product holder. The mixing body is to some degree controllable by means of the displacement device and can thereby be brought into cooperation with the additive dosing device. Thus no supplementary movable parts are required for output of the additive, and no electrical and/or mechanical connections are required between the product holder and the external environment for operation by a person or control device. Thus the measurements may continue without the need to open the product holder and without increasing the complexity of the measuring system. The reliability and reproducibility of the measurements of the parameter values of the foodstuff mixture thus also increase.

In the present invention, the foodstuffs concern fluid or viscous substances or products which are at least pumpable. Examples are simply water, but in particular milk or dairy products, food juices, fruit jellies, oils, sauces etc. In the context of the invention, the term "foodstuff" means a product which is eaten by another non-vegetable organism, such as by humans, animals and even microorganisms such as yeast or bacteria. Because these products are eaten or drunk, it is extremely important to know as many properties thereof as possible, in particular for products for human or animal consumption. Since they often also have a long shelf life, and/or a consumer packing thereof is used for a long time under widely varying conditions such as temperature, it is important to know the evolution of these properties over time. Such foodstuffs are often prepared by adding materials or additives to a base. It is important to know how the additive is absorbed in or reacts with base/foodstuff. Examples are products to be dissolved such as milk powder in water, coffee or tea, drinking chocolate powder in hot or cold milk, soup powder or stock cubes in water, the formation of an emulsion of oil and water etc. It may also be important to know how well a foodstuff resists the effect of a substance such as an enzyme or microorganism. The addition thereof at a specific moment without a chance of undesired addition of yet other substances or microorganisms gives reliable information on e.g. the stability of the foodstuff. The present invention may be useful in these and other situations because no human action is required for the addition of additive and the product holder may remain closed.

Also known are US2018/008080A1, that discloses an alternative solution with a spice hatch that can be opened by means of a permanent magnet, without requiring a physical connection of the main container, and US6166551A1, that deals with introducing a mixture of predetermined ingredients into a cooking vessel, but not with the present characterising portion.

Particular embodiments of the present invention are described in the appended dependent claims and will be explained in more detail below.

The mixing body is displaceable in the longitudinal direction of the product holder. This gives a better mixing than if the mixing body is simply rotated around an axis, as in known magnetic agitators. The displacement device for moving in the longitudinal direction may be configured in various ways. For example, the displacement device comprises a stack of at least two magnetic coils which can be controlled separately by the control device and are arranged around the product holder, as described specifically in WO2021066646-A1. The magnetic field induced by the one or more magnetic coils can move the mixing body by attracting or repelling this. This embodiment not only contains no moving parts but is also easy to adjust with respect to intensity of the field and can be switched on quickly. The displacement device may alternatively also comprise one or more external permanent magnets which are movable along a section. For this, the displacement device comprises a mechanical spindle or a compressed air or hydraulic device etc. One advantage of such embodiments is that these do not generate heat, or generate much less heat than coils, which heat may have a disruptive effect on the content of the product holder. The magnetic force for displacement can also be adapted by exchanging one or more magnets.

In general, the additive dosing device comprises a holder, cylinder or similar, referred to below as a vessel for simplicity. The vessel is here configured to receive the additive and may for example be filled outside the product holder before measurement, for example under sterile conditions, under a protective atmosphere etc. Then under control by the control device, the mixing body may be displaced to act on the additive vessel for output of the additive. The output is thus automatic.

For example, the additive dosing device comprises a closing means to be operated, in particular opened, by the mixing body. Such a closing means serves to keep the additive vessel, or at least the additive dosing device in general, closed until the controller commands the output. The closing means is not specifically restricted and may for example be selected depending on the properties of the additive, e.g. a powder or liquid.

In particular, the closing means is provided with a first coupling means, wherein the mixing body comprises a second coupling means which is configured to act on the first coupling means, wherein the control device is configured to move the mixing body such that the first coupling means and the second coupling means couple, and then move the mixing body away from the additive dosing device in order to release the additive. The coupling means creates a coupling between the mixing body and at least a part of the additive dosing device. Thus the part of the additive dosing device can be operated by the control device by means of the displacement device.

The coupling means may take various forms. In particular, the first coupling means and second coupling means each comprise a permanent magnet. The first coupling means is arranged in the closing means, in particular the removable cover, and the second coupling means in the mixing body. In many embodiments, the mixing body already comprises such a permanent magnet because of the movability under the influence of the magnet (magnetic fields) of the displacement device. Note however that alternatively, it is possible to make the second coupling means in the mixing body of magnetisable material, so that a coupling with the first coupling means can also be easily interrupted. Also note that a permanent magnet in the closing means is in principle also influenced by magnetic fields of the mixing device. These fields however often need not be very strong and in addition are often substantially homogenous. In contrast, the attraction force and hence the resulting coupling force between the first and second coupling means is very strong, depending on the mutual spacing. Thus in practice it is possible to set firstly the strength of the magnet of the first coupling means, and secondly the retaining force with which the closing means is initially held in place sufficiently large that movement of the mixing body by means of the mixing device does not lead to release of the closing means. Conversely, the strength of the magnet of the second coupling means may in practice be selected sufficiently large so that, after coupling with the first magnet, in combination, sufficiently strong movement is achieved by the mixing device to overcome said retaining force of the closing means and thus release the additive.

In other embodiments, the first coupling means and the second coupling means each comprise a complementary part of a removable or permanent snap connection. Here it is sufficient if the mixing body is pressed by the mixing device against the closing means, in particular the cover, and thereby the coupling created between the first and second coupling means. The snap connection may thus for example comprise elastic hooks or similar. Again, the magnetic strength of the magnetic material in the mixing body should be selected sufficiently large to overcome a retaining force with which the closing means is initially retained. Naturally, a coupling force between the first and second coupling means should not be greater.

In particular, the closing means comprises a flap to be opened by the mixing body. A flap may in this context be a hinged part for example, with a lock operable by the mixing body, such as a sliding pawl. Other possibilities are not excluded, such as a spring closure, such as the closure of a handle-less kitchen drawer.

Alternatively the closing means comprises a cover which is removable by the mixing body. If the mixing body is brought into contact with, in particular coupled with the cover by the control device, the control device may then move the combination of cover and mixing body away and open the additive vessel.

Also, the closing means may comprise a piston movable by the mixing body, wherein the vessel and/or the piston comprises an outlet opening via which the piston can press the additive out of the additive dosing device. An advantage of this is that the additive can be added in stages, for example by the mixing body moving the piston in stages. If no valve is provided in the additive dosing device and the opening through which additive is expelled is directed downward, the type of additive is often restricted to a substantially highly viscous fluid which does not flow out of the additive vessel purely under the influence of gravity, but this is also dependent on the cross-section of the opening. It is however also possible to provide the opening on a side or even a top of the additive vessel. Also, in all cases it is possible to provide a valve which closes the additive vessel but which allows the additive to pass through under the influence of the increased pressure in the vessel caused by the piston.

In some embodiments, the control device is configured to execute a mixing program via the mixing device, wherein the mixing body performs reciprocal movements at one more predetermined times and/or with a predefined speed and/or amplitude. Although it is possible in itself to allow the control device to receive and process external commands in order for the reciprocating movement to be performed by an operator, it is advantageous if this takes place automatically. Thus an entire protocol can be entered for performing a mixing, for example by moving the mixing body to and fro a predefined number of times at desired time intervals. Such a protocol may for example be dependent on the foodstuff in the product holder, the additive, the parameters to be measured etc.

The sensor device in the measuring system according to the invention may be of various types. For example, the sensor device may comprise one more of a camera, such as for detecting color, turbidity or sediment; a thermometer, useful for example because many reactions and variables are temperature-dependent; electrodes for measuring electrical variables such as conductivity or dielectric constant. In some embodiments, the sensor device comprises a viscosimeter for measuring viscosity. Viscosity is an important property of foodstuffs and is often dependent on time and/or temperature, but also often varies very greatly on addition of an additive. For example, the addition of soup powder to water, or a binder to a sauce. In a particularly attractive embodiment, the viscosimeter comprises a series of Hall sensors extending along the product holder for detecting magnetic signals, wherein the control device is configured to determine viscosity from the magnetic signals from the Hall sensors. Here, the mixing device sets the mixing body in motion by for example actuating magnetic coils in a suitable order, or by moving the external magnet in a predefined fashion, while the control device receives and processes the signals from the Hall sensors. The latter may be used to derive the speed of the mixing body, which in turn is an indication of the viscosity of the content of the product holder. For further explanation and details of this embodiment and for further features thereof, express reference is made to WO2021066646-A1. It is noted here that the embodiment described in the present invention is particularly attractive since the magnet of the mixing body can perform a threefold function. Firstly, the mixing body may be suitably moved through the foodstuff in the product holder in order to mix this with itself or with an additive. Secondly, the mixing body may serve to determine the viscosity of the foodstuff. Finally, by the action of the mixing body, additive may be output from an additive vessel so that the additive can be added to the foodstuff, so that further sampling can be performed. This may all take place without the need to open the product holder and also without potentially fault-susceptible mechanisms or connections to the external environment.

The invention will now be explained in more detail with reference to some non-restrictive exemplary embodiments and the drawing. In the drawing:
- Figures 1A, 1B and 1C show a measuring system 1 according to the invention in three stages during use, in schematic cross-section; and
- Figures 2A-D show, in schematic views, alternative additive dosing devices.

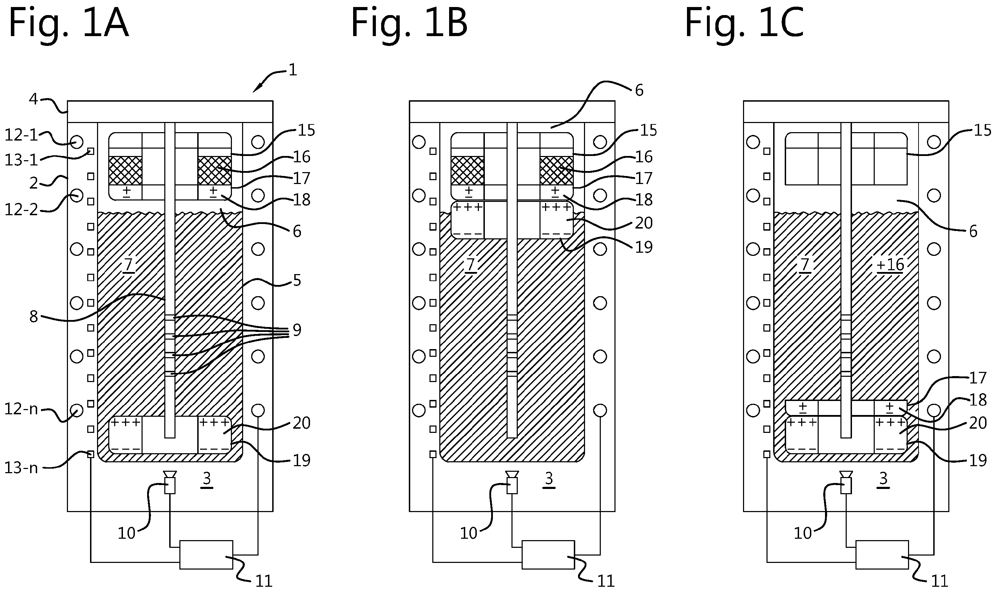

Figures 1A, 1B and 1C show a measuring system 1 according to the invention in three stages during use, in schematic cross-section, and in particular show a measuring system with a housing 2, a receiving space 3 and a cover 4. A product holder 5 has a product space 6 with a foodstuff 7 therein. Reference 8 indicates a probe with four electrodes 9 which are connected, as is a camera 10, to a control device 11.

References 12-1, 12-2,... 12-n indicate magnetic coils, and 13-1 to 13-n indicate Hall sensors. An additive vessel 15 contains an additive 16 and is closed by a cover 17 having a first magnet 18. A mixer 19 has a second magnet 20.

The measuring system 1 shown here has a housing 2 with a receiving space 3 for only a single product holder 5. It is quite possible to provide a larger housing and receiving space for multiple product holders. Since such a larger number makes no substantial difference to the invention, no further attention will be here paid to the larger number.

The product holder 5 is usually made of glass, for example for better perception of the contents such as with the camera 10, and because of favorable properties of many glass types, such as chemical resistance. Nonetheless, other materials such as stainless steel or similar are not excluded, wherein the camera is naturally no longer provided. The product holder 5 is here largely filled with a foodstuff 7. The foodstuff is a fluid or viscous foodstuff such as a dairy product, fruit juice etc., but may also be water.

The additive vessel 15 contains an additive 16, such as a powder which must be dissolved in the foodstuff 7, or a substance which must provoke or simulate a reaction such as ripening or perishing, such as a bacteria or mold culture etc. Examples are instant dessert powders, soluble coffee or tea, milk powder etc. For such additives, the dissolution behavior is important: how quickly and how well it dissolves, whether lumps are formed, the temperature dependence etc. Alternatively, it is useful to know how the foodstuff reacts to the added substance or culture: does perishing occur, and if so, which and how strongly etc.

The present invention provides the output of additive from an additive vessel 15. In this example, the vessel comprises a cover 17 with a (first) magnet 18. In figure 1A, the mixing body 19 is at the bottom and the cover 17 closes the additive vessel 15. This closure may be achieved for example in that a (weak) mechanical connection exists between cover and vessel etc. The magnets 18 and 20 in this embodiment have an annular shape and different strength, here indicated by the number of plus and minus signs, respectively the north and south poles. For the purpose of opening the additive vessel 15, the mixing body 19 is moved upward by targeted activation of magnetic coils 12-1 to 12-n. In figure 1B therefore, the mixing body 19 has moved upward and now lies against the cover 17. The stronger second magnet 20 lies much closer to the first magnet 18 than the first magnet 15, which is also weaker. If now the magnetic coils 12-1 to 12-n are again suitably energised so as to move the mixing body 19 downward, the mixing body 19 can pull the cover 17 with it and thus open the additive vessel 15, so that the additive 16 is released and can be mixed with the foodstuff 7. The latter situation shown in figure 1C.

Measurements on the foodstuff 7 - with or without the additive 16 - can now be carried out e.g. by a sensor, such as the optional electrodes 9 on the likewise optional measuring probe 8. These electrodes may for example in pairs measure the real and imaginary parts of the impedance of the foodstuff, which in itself is a known technique. Also, the optional camera 10 may perform optical measurements, such as with respect to turbidity and/or color of the foodstuff. If required, a light source may be provided (not shown here). Other sensors are also possible. One important example is a Hall sensor device comprising several Hall sensors, here indicated with reference signs 13-1 to 13-n, wherein the larger the number n between 8 and 20, the finer the resolution of the detection or monitoring. The Hall sensors 13 detect a magnetic field and also when this moves. Thus the Hall sensors 13 can detect whether and how the mixing body 19 moves. This information can be used in more than one way, for example by the mixing body 19, as will be explained below.

The mixing body 19 may be helpful for dissolving the additive 16. The additive may be added to the foodstuff 7 from the additive vessel 15 in many ways. One of these ways is discussed below. The mixing body moves a magnet 20 via which the mixing body, using the magnetic coils 12-1 to 12-n which are individually controllable by the control device 11, can be moved up and down, namely in the manner already described in WO 2021066646-A1. In fact the movement from figure 1A to figure 1B to figure 1C may be repeated one or more times. It is also possible to apply a different amplitude of the movements of the mixing body 19, by for example not energising the top magnetic coil(s). This naturally leads to mixing of the added additive 16 in the foodstuff 7.

Mixing often takes place following a fixed protocol in order to obtain good reliable and reproducible measurement values. The present invention is very suitable for this since firstly the product holder 5 need not be opened, no human actions are required and mixing itself takes place fully automatically, thereby also highly reproducibly. Such a protocol may for example comprise moving the mixing body 19 to and fro a predefined number of times over a predefined distance and possibly with a predefined speed. If required, this or an adapted mixing action can later be repeated one or more times. This is simple to perform by corresponding programming of the control device 11.

The mixing can be coupled to various situations by the control device 11, also external to the mixing. Firstly, a mixing action can be carried out prior to the release of the additive 16 from the vessel 15. This may for example serve to homogenise the foodstuff, which is often a complex assembly of many ingredients, so as not to negatively influence the following mixing. If desired, also one or more mixing actions can be carried out in advance so as to better guarantee the condition of the foodstuff, for example while this matures or ages. For example, the control device may be configured to measure one or more parameter values in the foodstuff 7 or mixture 7 + 16 prior to, during and/or after mixing.

The measuring system according to the invention furthermore provides for checking the position of the mixing body 19 by means of the second magnet 20 and the series of Hall sensors 13-1 to 13-n. As described above, by means of the Hall sensors actively connected thereto, the control device may determine the position of the second magnet 20 and hence the mixing body 19. By repeated detection, also any movement and speed of the mixing body can be detected. Thus the control device 11 can monitor whether the planned mixing using the mixing body 19 has actually taken place.

Then via the mixing body 19, the separately energisable magnetic coils 12 and the Hall sensors 13, the control device 11 can also determine viscosity values of the foodstuff. For this, use may be made of the technique described in the above-mentioned document WO2021/066646. In brief, the control device 11 moves the mixing body through the foodstuff by means of the magnetic coils 12, and collect the signals measured by the Hall sensors 13 during the movement. In particular, the control device can determine the displacement speed of the mixing body from the movement speed of the peak of the signals. Since it is also known how strongly the magnetic coils attract the mixing body 19, and the form of the mixing body is established and known, the friction on the mixing body can thereby be determined. The control device 11 can then determine the viscosity of the foodstuff (with additive where applicable) in the known fashion. It is important to note that by means of the present invention, a functionality is added without an increase in mechanical complexity.

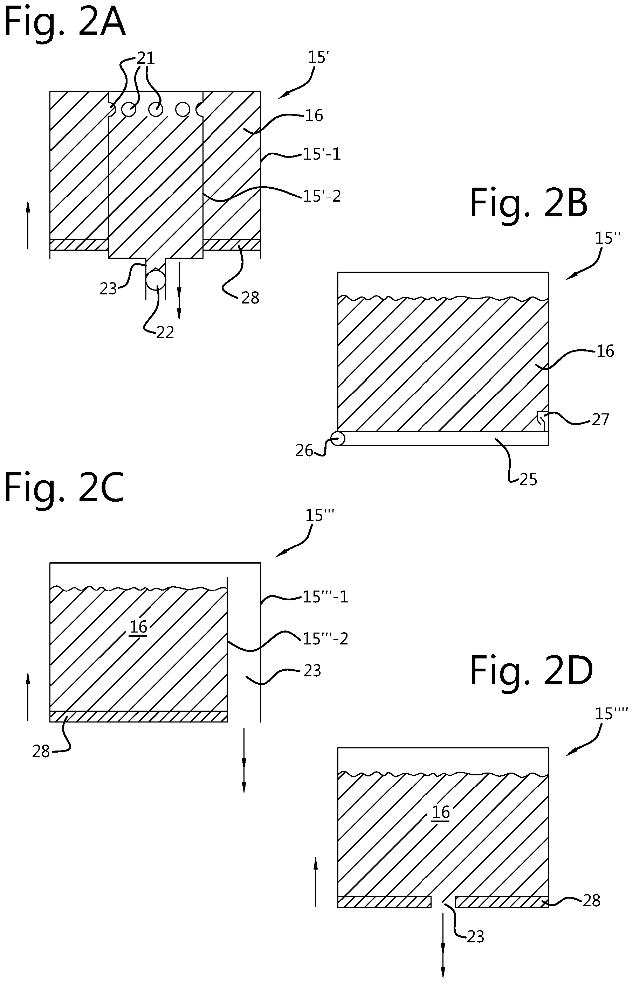

Figures 2A-D shows schematically some alternative additive dosing devices indicated by 15', 15" and 15‴ respectively.

Figure 2A shows a schematic cross-sectional view of an alternative additive dosing device 15', with an outer wall 15'-1 and a concentric inner wall 15'-2. An additive 16 is located therein. Reference sign 28 indicates the piston and 21 through-flow holes. 22 indicates a one-way valve and 23 an outlet.

The piston is here shown as an annular body in the housing of the additive dosing device 15', and extends between the outer wall 15'-1 and the inner wall 15'-2. The piston can be pushed in the direction of the single arrow. On such a movement, the piston expels the content of the dosing device 15' through the through-flow holes 21 and the outlet 22.

If the control device is to add additive, it activates the series of magnetic coils to raise the mixing body, which thereby pushes hits the piston 28 and pushes this upward. In this way, the piston 20 presses the additive 16 out through the one-way valve 22 via the outlet 23 in the direction of the double arrow. The additive 16 then reaches the foodstuff (not shown) and can be mixed in. This embodiment also requires no human actions. A further advantage is that the mixing body can move the piston in stages in order thus to add the additive 16 to the foodstuff in stages.

It is noted here that it may be advantageous to give the inner wall 15'-2 a much smaller radius than the outer wall 15'-1, so that the piston 20 can expel a larger part of the additive 16.

This embodiment is suitable for example for liquid or otherwise pumpable additives 16, such as an oil or similar. The one-way valve 22 is selected depending on the properties of the additive. Thus for a relatively highly viscous oil, a single check valve or duck-bill will be sufficient to keep the additive 16 in the device 15' until the piston 28 is operated. In the case of very high viscosity, the valve 22 may even be omitted. For a very low viscosity oil or similar, however, a controllable butterfly valve (e.g. rotatable by upward movement of the mixing body) or similar may be used.

Alternatively, the outlet 23 may also be attached to a side or even top of the outer wall 15'-1, namely on the top, i.e. on or close to the side which is remote from the piston 28 in the starting situation. Thus the piston 28 may be configured as a closed disc which covers the entire cross-section of the dosing device 15', so that neither the inner wall 15'-2 nor the through-flow holes 21 are required.

According to figure 2C, it is possible to configure the dosing device 15' asymmetrically with a large piston 28 and next to this a narrow outlet 23. An advantage of this embodiment is that a very large amount of the additive 16 can be expelled, and fundamentally no closing means such as a flap is required. A disadvantage may be that this is not symmetrical and the mixing body must thus be properly guided such as with guide surfaces, and in this embodiment it is more difficult to carry out measurements by means of a measurement probe, such as probe 8 in figure 1.

Again alternatively, figure 2D shows an additive dosing device 15‴ with a piston 28 on which the outlet 23 is situated. Thus a symmetrical design is obtained which does not have guide problems for the mixing body. Also, in this embodiment no valve is shown in the outlet 23. Thus this embodiment can only be used for highly viscous fluids. If a one-way valve is arranged in the outlet 23, this is fundamentally suitable for all fluids.

Yet an alternative additive dosing device 15", indicated schematically in figure 2B, again contains an additive 16. A cover 25 pivots around a pivot axis 26. Reference 27 indicates a locking device.

In this embodiment, the control device can unlock the additive dosing device 15" as follows. In the starting state, the cover 25 is locked by means of the locking device 27. The latter comprises for example two cooperating coupling means, such as spring hooks, which can be separated from one another relatively easily.

The cover 25 is here made of or contains magnetisable material such as iron. When the control device now moves the mixing body, containing a magnet, against the cover 25, the cover also becomes magnetic under the influence of the magnetic field of said magnet. Also the magnetic field of one or more of the magnetic coils also induces magnetism in the cover 25, but weaker. In any case, the mixing body can now attract the cover 25 and move this downward when the control device operates the magnetic coils to move the mixing body downward. The magnetic strength, the locking device 27 and similar should be dimensioned and configured such that the magnetic coils can exert an attraction force on the mixing body plus cover which is sufficiently large to unlock the locking device 27. Carried by the mixing body, the cover 25 will pivot around the pivot axis 26, whereupon gravity will discharge the additive. Thus the additive dosing device 15" can release the additive 16 without physical connection to the control device and without actively moving parts. Alternatively, the cover 25 may also be provided with a permanent magnet. This may make the function more reliable.

## Claims

1. A measuring system (1) for automatically determining a quality of a foodstuff mixture (7, 16), and comprising
- at least one product holder (5) with a longitudinal direction for receiving a fluid or viscous foodstuff (7),
- a housing (2) with a receiving space (3) for receiving the at least one product holder,
- a sensor device (8, 9, 10, 13) for measuring a parameter value of the foodstuff in the product holder, and
- a control device (11) for controlling the measuring system and processing the measured parameter values,
furthermore comprising
and
- a mixing device which comprises:
- a mixing body (19) arranged in the product holder and comprising a magnetisable or magnetic material, and
- a displacement device (12) arranged outside the product holder for displacing the mixing body along the longitudinal direction,
**characterised, in that** the measuring system further comprises an additive dosing device (15, 17) which is configured to output an additive (16) to the foodstuff in the product holder under control by the control device when the product holder is closed,
wherein the mixing device is configured to act on the additive dosing device via said mixing body for output of the additive.

2. The measuring system as claimed in claim 1, wherein the additive dosing device comprises a vessel (15) with closing means (17; 25**;** 28) to be operated by the mixing body (19).

3. The measuring device as claimed in claim 2, wherein the closing means is provided with a first coupling means (18) , wherein the mixing body comprises a second coupling means (20) which is configured to act on the first coupling means, wherein the control device is configured to move the mixing body such that the first coupling means and the second coupling means couple, and then move the mixing body away from the additive dosing device in order to release the additive.

4. The measuring device as claimed in claim 3, wherein the first coupling means (18) and the second coupling means (20) each comprise a permanent magnet or magnetisable material, or a complementary part of a removable or permanent snap connection.

5. The measuring device as claimed in claim 2, 3 or 4, wherein the closing means comprises a flap (17) to be opened by the mixing body or a removable cover (25).

6. The measuring device as claimed in claim 2, 3 or 4, wherein the closing means comprises a piston (28) movable by the mixing body, and wherein the vessel and/or the piston comprises an outlet opening (23) via which the piston can press the additive out of the additive dosing device.

## Patentansprüche

1. Messsystem (1) zum automatischen Bestimmen einer Qualität einer Lebensmittelmischung (7, 16), umfassend
- mindestens einen Produkthalter (5) mit einer Längsrichtung zum Aufnehmen eines flüssigen oder viskosen Lebensmittels (7),
- ein Gehäuse (2) mit einem Aufnahmeraum (3) zum Aufnehmen des mindestens einen Produkthalters,
- eine Sensorvorrichtung (8, 9, 10, 13) zum Messen eines Parameterwertes des Lebensmittels im Produkthalter und
- eine Steuervorrichtung (11) zum Steuern des Messsystems und Verarbeiten der gemessenen Parameterwerte,
und ferner umfassend
- eine Mischvorrichtung, die umfasst:
- einen im Produkthalter angeordneten Mischkörper (19), der ein magnetisierbares oder magnetisches Material umfasst, und
- eine außerhalb des Produkthalters angeordnete Verschiebevorrichtung (12) zum Verschieben des Mischkörpers entlang der Längsrichtung,
**dadurch gekennzeichnet, dass** das Messsystem ferner eine Zusatzstoff-Dosiervorrichtung (15, 17) umfasst, die so konfiguriert ist, dass sie unter Steuerung durch die Steuervorrichtung einen Zusatzstoff (16) an das Lebensmittel im Produkthalter abgibt, wenn der Produkthalter geschlossen ist,
wobei die Mischvorrichtung so konfiguriert ist, dass sie über den Mischkörper auf die Zusatzstoff-Dosiervorrichtung zur Abgabe des Zusatzstoffes wirkt.

2. Messsystem nach Anspruch 1, wobei die Zusatzstoff-Dosiervorrichtung einen Behälter (15) mit Verschlussmitteln (17; 25; 28) umfasst, die durch den Mischkörper (19) betätigt werden sollen.

3. Messvorrichtung nach Anspruch 2, wobei das Verschlussmittel mit einem ersten Kopplungsmittel (18) versehen ist, wobei der Mischkörper ein zweites Kopplungsmittel (20) umfasst, das so konfiguriert ist, dass es auf das erste Kopplungsmittel wirkt, wobei die Steuervorrichtung so konfiguriert ist, dass sie den Mischkörper so bewegt, dass das erste Kopplungsmittel und das zweite Kopplungsmittel gekoppelt werden, und dann den Mischkörper von der Zusatzstoff-Dosiervorrichtung wegbewegt, um den Zusatzstoff freizusetzen.

4. Messvorrichtung nach Anspruch 3, wobei das erste Kopplungsmittel (18) und das zweite Kopplungsmittel (20) jeweils einen Permanentmagneten oder ein magnetisierbares Material oder einen komplementären Teil einer lösbaren oder permanenten Schnappverbindung umfassen.

5. Messvorrichtung nach Anspruch 2, 3 oder 4, wobei das Verschlussmittel eine durch den Mischkörper zu öffnende Klappe (17) oder eine lösbare Abdeckung (25) umfasst.

6. Messvorrichtung nach Anspruch 2, 3 oder 4, wobei das Verschlussmittel einen durch den Mischkörper bewegbaren Kolben (28) umfasst und wobei der Behälter und/oder der Kolben eine Auslassöffnung (23) umfasst, über die der Kolben den Zusatzstoff aus der Zusatzstoff-Dosiervorrichtung drücken kann.

## Revendications

1. Système de mesure (1) permettant de déterminer automatiquement la qualité d'un mélange alimentaire (7, 16), et comprenant
- au moins un réceptacle à produit (5) ayant une direction longitudinale, destiné à recevoir un aliment fluide ou visqueux (7),
- un boîtier (2) ayant un espace de réception (3) destiné à recevoir au moins un réceptacle à produit,
- un dispositif de capteur (8, 9, 10, 13) destiné à mesurer une valeur de paramètre de l'aliment dans le réceptacle à produit, et
- un dispositif de commande (11) destiné à commander le système de mesure et traiter les valeurs de paramètres mesurées,
et comprenant en outre
- un dispositif de mélange qui comprend :
- un corps de mélange (19) disposé dans le réceptacle à produit et comprenant un matériau magnétisable ou magnétique, et
- un dispositif de déplacement (12) disposé à l'extérieur du réceptacle à produit, destiné à déplacer le corps de mélange le long de la direction longitudinale,
**caractérisé en ce que** le système de mesure comporte, en outre, un dispositif de dosage d'additif (15, 17) configuré pour distribuer un additif (16) dans l'aliment dans le réceptacle à produit sous la commande du dispositif de commande lorsque le réceptacle à produit est fermé,
dans lequel le dispositif de mélange est configuré pour agir sur le dispositif de dosage d'additif par l'intermédiaire dudit corps de mélange pour la distribution de l'additif.

2. Système de mesure selon la revendication 1, dans lequel le dispositif de dosage d'additif comprend un récipient (15) avec un moyen de fermeture (17, 25, 28) destiné à être actionné par le corps de mélange (19).

3. Dispositif de mesure selon la revendication 2, dans lequel le moyen de fermeture est pourvu d'un premier moyen d'accouplement (18), dans lequel le corps de mélange comprend un second moyen d'accouplement (20) configuré pour agir sur le premier moyen d'accouplement, dans lequel le dispositif de commande est configuré pour déplacer le corps de mélange de façon à ce que le premier moyen d'accouplement et le second moyen d'accouplement s'accouplent, puis écarter le corps de mélange du dispositif de dosage d'additif afin de libérer l'additif.

4. Dispositif de mesure selon la revendication 3, dans lequel le premier moyen d'accouplement (18) et le second moyen d'accouplement (20) comprennent chacun un aimant permanent ou un matériau magnétisable, ou une partie complémentaire d'une liaison à emboîtement-pression amovible ou permanente.

5. Dispositif de mesure selon la revendication 2, 3 ou 4, dans lequel le moyen de fermeture comprend un clapet (17) destiné à être ouvert par le corps de mélange ou un couvercle amovible (25).

6. Dispositif de mesure selon la revendication 2, 3 ou 4, dans lequel le moyen de fermeture comprend un piston (28) déplaçable par le corps de mélange, et dans lequel le récipient et/ou le piston comprennent une ouverture de sortie (23) par laquelle le piston peut expulser l'additif hors du dispositif de dosage d'additif.
